# EUROPEAN PATENT SPECIFICATION

(11) **EP 3 027 011 B1**
(45) Date of publication and mention of the grant of the patent: **04.07.2018**
(21) Application number: 14747165.0
(22) Date of filing: 22.07.2014
(51) Int. Cl.: G01N 15/06, G01N 1/40, G01N 33/04

(54) **A METHOD OF PREPARING A MILK SAMPLE, AND A DEVICE CONFIGURED TO BE USED WHEN PREPARING A MILK SAMPLE**
VERFAHREN ZUR HERSTELLUNG EINER MILCHPROBE UND VORRICHTUNG ZUR VERWENDUNG BEI DER ZUBEREITUNG EINER MILCHPROBE
PROCÉDÉ DE PRÉPARATION D'UN ÉCHANTILLON DE LAIT, ET DISPOSITIF CONÇU POUR ÊTRE UTILISÉ LORS DE LA PRÉPARATION D'UN ÉCHANTILLON DE LAIT

(30) Priority: 29.07.2013 SE 1350917
(43) Date of publication of application: 08.06.2016
(73) Proprietor: DeLaval Holding AB, 147 21 Tumba (SE)
(72) Inventor: FOLKESSON, Johan, S-147 21 Tumba (SE); JÖNSSON, Patrik, S-147 21 Tumba (SE); NILSSON, Lars, S-147 21 Tumba (SE); PERSSON, Örjan, S-147 21 Tumba (SE)
(74) Representative: Lilliehorn, Tobias
(86) International application number: PCT/SE2014/050901
(87) International publication number: WO 2015/016763

(56) References cited:
- EP-A2- 0 342 501
- EP-A2- 2 515 094
- WO-A1-92/02632
- CN-A- 102 719 426
- RU-C2- 2 473 896
- SU-A1- 1 654 739
- US-A1- 2010 105 083
- US-A1- 2012 003 626
- 'MILK AND MILK PRODUCTS. SAMPLING AND PREPARATION OF SAMPLES FOR TESTING (STANDARDINFORM)' GOST 2009, MOSCOW, pages 3622 - 3668

## Description

### TECHNICAL FIELD OF THE INVENTION

The present invention refers generally to the preparation of milk samples for preparing the milk for further analysis with respect to the quality of the milk, especially the number and kind of somatic cells in the milk. More precisely, the present invention refers to a method for preparing a milk sample according to the preamble of claim 1. The invention also refers to a device configured to be used when preparing a milk sample.

### BACKGROUND OF THE INVENTION AND PRIOR ART

In the prior art, it is known to estimate the number of somatic cells in milk extracted from an animal in order to establish whether the animal suffers from any disease, such as mastitis. For obtaining such an estimate, a milk sample is taken from the extracted milk. The milk sample is prepared before being analysed in order to facilitate counting of somatic cells in the milk.

US-6,979,550 discloses a method for detection and prediction of mastitis. The method comprises the steps of determining the value of mastitis indicators and comparing the values with predetermined standards, wherein deviation from the standards provides a measure of mastitis.

It is known to centrifuge a milk sample in connection with preparing the sample for analysis, compare for example WO92/02632, US2010/0105083, CN102719426 and EP0342501.

EP2515094 discloses that the milk samples are mixed by a mixer in the vicinity of the bottom of the sample reservoir, preferably by utilising a membrane agitating the milk.

In the milk sample, containing fat and casein, it is difficult to identify and observe somatic cells, especially in an optical manner through a microscope. According to the prior art, the preparation therefore comprises the addition of various chemicals to the milk sample to react with the somatic cells in order to make the somatic cells observable. The addition of chemicals, make the preparation difficult and time consuming.

These kind of chemicals can also affect the somatic cells, making it difficult to identify which types of somatic cell are contained in the milk sample. Moreover, the milk sample has to be taken care of after the analysis, which is a problem when the milk sample is contaminated with chemicals of various kind.

### SUMMARY OF THE INVENTION

The object of the present invention is to provide an improved method and an improved device to be used when preparing a milk sample for being analysed. In particular, it is aimed at a method and a device enabling preparation of a milk sample without addition of chemicals reacting with somatic cells in the milk sample.

This object is achieved by the method as defined in claim 1. The invention permits to obtain a sample mixture, which comprises the solution and the remaining sample part, e.g. the first sample part or the second sample part. In case the remaining sample part is formed by the second sample part, the sample mixture constitutes a representative quantity of the milk extracted from the animal with respect to the content of the second constituent, such as somatic cells, wherein the solution is mixed with the second sample part. In other words, the invention permits to obtain a milk sample which contains the same percentage of somatic cells as the milk extracted from the animal. The sample mixture so obtained is thus suitable for being discharged and forwarded to an analysing equipment for analysing the milk, especially for optical counting of the number of somatic cells in a microscope. Furthermore, the invention permits somatic cells of various types in the sample mixture to be recognised and counted optically in a microscope. The somatic cells have not been negatively affected by any chemicals, which possibly could have prevented identification of the different types of somatic cells contained in the extracted milk and in the milk sample. Thus, the invention enables identification of each type of somatic cells, such as lymphocytes, epithelial cells, neutrophils, monocytes, basophils, macrophages, etc.

The invention also permits to obtain a sample mixture which constitutes a representative quantity of the milk extracted from the animal with respect to the content of the first constituent, such as fat, wherein the solution is mixed with the first sample part being the remaining sample part. The sample mixture so obtained is suitable for being discharged to an analysing equipment for analysing and/or counting the fat cells of the milk.

According to an aspect of the invention, the solution comprises or consists of a biologically acceptable saline solution. In particular, the solution may comprise or consist of a biological acceptable NaCI-solution. The salt content may then correspond to the salt content of the body liquid of the animal. Such a saline solution will not affect the somatic cells.

According to a further aspect of the invention, the first volume is greater than the second volume. Advantageously, the second volume may be less the 10% of the first volume. Moreover, the second volume may be more than 1% of the first volume.

According to the invention, the step of providing the milk sample is followed by a step of isolating the milk sample in a space formed by a first fill chamber, defining the first volume, and a second fill chamber, defining the second volume.

According to the invention, the step of providing the solution is followed by a step of isolating the solution in a space formed by a first mixing chamber, defining a volume equal to the first volume, and a second mixing chamber, defining a volume equal to the second volume, wherein the step of removing one of the sample parts comprises displacing at least one of the second fill chamber and the first mixing camber to communicate with each other. Advantageously, the second fill chamber and the first mixing chamber are then align with each other so that the second sample part in the second fill chamber may flow into the first mixing chamber, and the solution in the first mixing chamber may flow into the second fill chamber. The mixing step may then be efficiently performed.

According to a further aspect of the invention, the step of mixing is performed with the aid of a stirring member. According to one embodiment, the stirring member may be provided in the first mixing chamber, in the case when the second sample part, i.e. the second constituent, is to be mixed with the solution. The stirring member may then be movable in the first mixing chamber and into the second fill chamber. According to another embodiment, the stirring member may be provided in the second mixing chamber, in the case when the first sample part, i.e. the first constituent, is to be mixed with the solution. The stirring member may then be movable in the second mixing chamber and into the first fill chamber.

According to a further aspect of the invention, the discharging step comprises transferring a predetermined portion of the sample mixture to an analysing equipment configured for counting and analysing the cell content. The predetermined portion will contain the same percentage of somatic cell, or alternatively fat cells, as the milk sample, and thus as the milk extracted from the animal. In either case, the somatic cells or the fat cells are uniformly distributed in the predetermined portion.

According to a further aspect of the invention, the first constituent is fat and the second constituent is somatic cells. The milk may also contain a third constituent, which may be casein. In case the sample mixture contains somatic cells and the solution, the third constituent may together with the first constituent be contained in the first sample part. Correspondingly, in case the sample mixture contains fat cells and the solution, the third constituent may together with the second constituent be contained in the second sample part.

The object is also achieved by the device initially defined, which comprises a plurality of sections including at least
a first section forming a first communication passage extending through the first section,
a second section forming a first fill chamber, defining a first volume and extending through the second section, and a first mixing chamber, defining a volume equal to the first volume and extending through the second section,
a third section forming a second fill chamber, defining a second volume and extending through the third section, and a second mixing chamber, defining a volume equal to the second volume and extending through the third section, and
a fourth section forming a second communication passage extending through the fourth section,
wherein at least the second section and the third section are individually movable transversely to a longitudinal axis of the device to permit different relative positions of the sections, all sections being arranged after each other along the longitudinal axis.

Such a device is suitable for being used when performing the method defined in claim 1. The sections being transversely movable may thus be moved to various positions enabling to obtain the sample mixture. Consequently, the device according to the invention is suitable for receiving a milk sample and for being used to produce a sample mixture, which constitutes a representative quantity of said milk extracted from the animal with respect to the content of somatic cells, i.e. a sample mixture that contains the same percentage of somatic cells as said milk extracted from the animal. In particular, the sample mixture may be achieved by using the method discussed above, especially by subjecting the device to the method steps of claim 1. As mentioned above, the sample mixture is suitable for being discharged and forwarded to an analysing equipment for analysing the milk, especially for counting the number of different somatic cells in an optical manner. According to the explanations given above, the device is also suitable for obtaining a sample mixture permitting analysis of the fat cells of the milk.

According to an embodiment of the invention the second section and the third section are individually movable transversely to the longitudinal axis to said relative positions, which define different communications possibilities between the first communication passage, the first fill chamber, the first mixing chamber, the second fill chamber, the second mixing chamber, and the second communication passage.

According to a further embodiment of the invention, the first section, the second section, the third section and the fourth section are configured to permit the following consecutive relative positions:
a first position, in which the first communication passage, the first fill chamber, the second fill chamber and the second communication passage communicate with each other,
a second position, in which the first fill chamber and the second fill chamber are communicating with each other and closed to the first communication passage and the second communication passage,
a third position, in which the first communication passage, the first mixing chamber, the second mixing chamber and the second communication passage communicate with each other,
a fourth position, in which either the first mixing chamber and the second fill chamber or first fill chamber and the second mixing chamber are communicating with each other and closed to the first communication passage and the second communication passage, and
a fifth position, in which the first communication passage and the second communication passage communicate with each other and with either the first mixing chamber and the second fill chamber or first fill chamber and the second mixing chamber.

The first position is suitable for performing the step of providing a milk sample. The second position is suitable for performing the centrifuging of the milk sample. The third position is suitable for providing the solution. The fourth step is suitable for mixing the second sample part or the first sample part with the solution. The fifth step is suitable for discharging the sample mixture.

According to a further embodiment of the invention,
the first position is configured to permit supply of a milk sample containing at least a first constituent and a second constituent to the first fill chamber and the second fill chamber,
the second position is configured to permit centrifuging of the milk sample contained in the first fill chamber and the second fill chamber to obtain a first sample part of a first volume, in which first sample part the first constituent is contained, and a second sample part of a second volume, in which second sample part the second constituent is contained,
the third position is configured to permit supply of a solution to the first mixing chamber and the second mixing chamber,
the fourth position is configured to permit mixing of the solution and either the second sample part, in the first mixing chamber and the second fill chamber to form a sample mixture in which the second sample part is distributed, or the first sample part, in the first fill chamber and the second mixing chamber to form a sample mixture in which the first sample part is distributed, and
the fifth position is configured to permit discharge of the sample mixture.

According to a further embodiment of the invention, the second communication passage comprises a primary channel and a secondary channel. Advantageously, the first communication passage, the first fill chamber and the second fill chamber may communicate with the primary channel in the first position for the supply of the milk sample to the first fill chamber and the second fill chamber. Moreover, the first communication passage, the first mixing chamber and the second mixing chamber may communicate with the primary channel in the third position for the supply of the solution to the first mixing chamber and the second mixing chamber. Still further, the first communication passage and either of the first mixing chamber and the second fill chamber or the first fill chamber and the second mixing chamber may communicate with the secondary channel in the fifth position for the discharge of the sample mixture.

According to a further embodiment of the invention, the first fill chamber and the second fill chamber extend in parallel with a longitudinal axis of the device, wherein the first mixing chamber and the second mixing chamber extend in parallel with said longitudinal axis.

According to a further embodiment of the invention, the first section, the second section and the third section are individually movable by being rotatable around the longitudinal axis. Alternatively, the first section, the second section and the third section are individually movable by being linearly displaceable transversely the longitudinal axis.

According to a further embodiment of the invention, a first actuator is provided for moving the first section, a second actuator is provided for moving the second section, and a third actuator is provided for moving the third section. Advantageously, the first, second and third actuators may be individually controlled by means of a controller.

According to a further embodiment of the invention, the plurality of sections comprises a fifth section provided between the second section and the third section, and forming a fifth fill chamber, defining a fifth volume, and a fifth mixing chamber, defining a volume equal to the fifth volume. Thanks to such fifth sections is possible to separate the any one of the first, second and third constituents of the milk. The invention thus enable analysis of the content of each of the three constituents.

According to a further embodiment of the invention, the sections, are configured to permit the following consecutive relative positions:
a first position, in which the first communication passage, the first fill chamber, the second fill chamber, the fifth fill chamber and the second communication passage communicate with each other,
a second position, in which the first fill chamber, the second fill chamber and the fifth fill chamber are communicating with each other and closed to the first communication passage and the second communication passage,
a third position, in which the first communication passage, the first mixing chamber, the second mixing chamber, the fifth mixing chamber and the second communication passage communicate with each other,
a fourth position, in which one of the first, second and fifth fill chambers is communicating with two of the first, second and fifth mixing chambers, thereby forming a channel extending through the second, fifth and third sections and being closed to the first communication passage and the second communication passage, and
a fifth position, in which the first communication passage and the second communication passage communicate with each other and with said channel.

### BRIEF DESCRIPTION OF THE DRAWINGS

The present invention is now to be explained more closely through a description of various embodiments and with reference to the drawings attached hereto.
- Fig 1: shows a schematic view of a device for preparing a milk sample in a first position according to a first embodiment of the invention.
- Fig 2: shows a schematic view of four section of the device in Fig 1 in a second position.
- Fig 3: shows a schematic view of the four sections of the device in Fig 1 in a third position.
- Fig 4: shows a schematic view of the four sections of the device in Fig 1 in a fourth position.
- Fig 5: shows a schematic view of the four sections of the device in Fig 1 in a fifth position.
- Fig 6: shows a schematic view of a device for preparing a milk sample in a first position according to a second embodiment of the invention.
- Fig 7: shows a schematic perspective view of a device for preparing a milk sample according to a third embodiment of the invention.
- Figs 8A: shows a side view of the device in Fig 7 in a first position.
- Figs 8B: shows an end view of the device in Fig 8A.
- Figs 8C: shows a sectional view along the line A-A in Fig 8B.
- Figs 9A: shows a side view of the device in Fig 7 in a second position.
- Figs 9B: shows an end view of the device in Fig 9A.
- Figs 9C: shows a sectional view along the line B-B in Fig 9B.
- Figs 10A: shows a side view of the device in Fig 7 in an intermediate position.
- Figs 10B: shows an end view of the device in Fig 10A.
- Figs 10C: shows a sectional view along the line C-C in Fig 10B.
- Figs 11A: shows a side view of the device in Fig 7 in a third position.
- Figs 11B: shows an end view of the device in Fig 11A.
- Figs 11C: shows a sectional view along the line D-D in Fig 11B.
- Figs 12A: shows a side view of the device in Fig 7 in a fourth position.
- Figs 12B: shows an end view of the device in Fig 12A.
- Figs 12C: shows a sectional view along the line E-E in Fig 12B.
- Figs 13A: shows a side view of the device in Fig 7 in a fifth position.
- Figs 13B: shows an end view of the device in Fig 13A.
- Figs 13C: shows a sectional view along the line F-F in Fig 13B.
- Fig 14: shows a schematic view of a device for preparing a milk sample in a first position according to a fourth embodiment of the invention.

### DETAILED DESCRIPTION OF VARIOUS EMBODIMENTS OF THE INVENTION

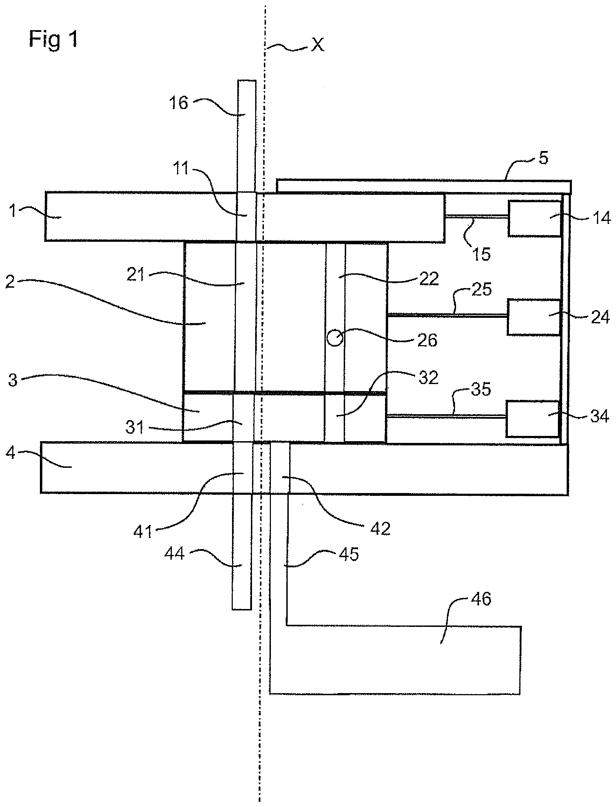

Fig 1 discloses a device, which is configured for being used when preparing a milk sample from milk extracted from an animal. The milk contains a plurality of different constituents, such as fat, casein and somatic cells. In the following description, the invention will be explained with respect to a first constituent being fat, and a second constituent being somatic cells. A third constituent may be casein.

An amount of somatic cells may always be present in the milk, but in case the animal suffers from a disease, such as mastitis, the number of somatic cells in the milk may be significant. It is important to be able to count the number of somatic cells, i.e. to enable the performance of a so called Somatic Cell Count, SCC, in number of cells per micro litre, and to be able to determine the different types of somatic cells in the milk.

The device comprises a first section 1, a second section 2, a third section 3 and a fourth section 4. The device defines a longitudinal axis X, which, at least in the first position disclosed in Fig 1, extends through all sections 1 to 4. The sections 1 to 4 are displaceable in relation to each other to take different positions as explained below. In the first embodiment, the first section 1, the second section 2 and the third section 3 are individually linearly displaceable transversely to the longitudinal axis X, whereas the fourth section 4 is attached and fixed to a frame 5. It is to be noted, that it is possible to make any one of the other sections 1, 2, 3 fixed in relation to the frame 5, wherein the fourth section 4 may be movable with respect to the frame 5.

A first actuator 14 is provided for moving the first section 1. The first actuator 14 is attached to the frame 5 and connected to the first section 1 via a schematically indicated first transmission device 15 or transmission element, such as a piston, a gear rack, a pulley etc.

A second actuator 24 is provided for moving the second section 2. The second actuator 24 is attached to the frame 5 and connected to the second section 2 via a schematically indicated second transmission device 25 or transmission element, such as a piston, a gear rack, a pulley etc.

A third actuator 34 is provided for moving the third section 3. The third actuator 34 is attached to the frame 5 and connected to the third section 2 via a schematically indicated third transmission device 35 or transmission element, such as a piston, a gear rack, a pulley etc.

The surfaces of each section 1 to 4, facing and adjoining another section 1 to 4, have a fine surface roughness ensuring abutment, or tight abutment, against the adjoining surface. The surfaces will thus function as seal surfaces.

The first section 1 forms a first communication passage 11 extending through the first section 1. In the first embodiment disclosed, the first communication passage 11 extends in parallel with the longitudinal axis X.

The second section 2 forms a first fill chamber 21, defining a first volume, and a first mixing chamber 22, defining a volume equal to the first volume. The first fill chamber 21 and the first mixing chamber 22 both extend through the second section 2 in parallel with the longitudinal axis X.

The third section 3 forms a second fill chamber 31, defining a second volume, and a second mixing chamber 32, defining a volume equal to the second volume. The second fill chamber 31 and the second mixing chamber 32 both extend through the third section 3 in parallel with the longitudinal axis X. The first volume is greater than the second volume. The second volume may be less the 10% of the first volume, and more than 1% of the first volume.

The fourth section 4 forms a second communication passage, which comprises a primary channel 41 and a secondary channel 42, which both extend through the fourth section 4. In the first embodiment disclosed, the primary channel 41 and a secondary channel 42 extend in parallel to the longitudinal axis X.

The first section 1, the second section 2, the third section 3 and the fourth section 4 are configured to permit the following consecutive relative positions.

### First position

In a first position, see Fig 1, the first communication passage 11, the first fill chamber 21, the second fill chamber 31 and the primary channel 41 communicate with each other. The first communication passage 11, the first fill chamber 21, the second fill chamber 31 and the second communication passage 43 are aligned with each other in the first position, thereby forming a channel extending through the device, in particular in parallel with the longitudinal axis X. It is to be noted, that the first communication passage 11 and the primary channel 41 do not necessarily need to extend straight through the first section 1 and fourth section 4, respectively, but may change direction in the respective section 1 and 4.

In the first position, the milk sample may thus be supplied to the first fill chamber 21 and the second fill chamber 31 via the primary channel 41. The primary channel 41 may be connected to an inlet conduit 44 and operate as an inlet to the first fill chamber 21 and the second fill chamber 31. The first communication passage 11 may be connected to an outlet conduit 16, so that the milk to be supplied may flow through the device, thereby ensuring that the first fill chamber 21 and the second fill chamber 31 are filled. The inlet conduit 44 may be connected to the milking equipment and receive milk from a milk collecting member (not disclosed) of the milking equipment.

During the supply of milk, the milk is thus flowing through the primary channel 41, the first fill chamber 21, the second fill chamber 31 and the first communication passage 11. When these are filled with milk and no gas bubbles are present, the second section 2 and the third section 3 are moved with the aid of the second actuator 24 and the third actuator 34 so that a second position is obtained. It is to be noted that the milk may flow also in the opposite direction during the supply of milk, i.e. in through the first communication passage 11 and out through the primary channel 41.

### Second position

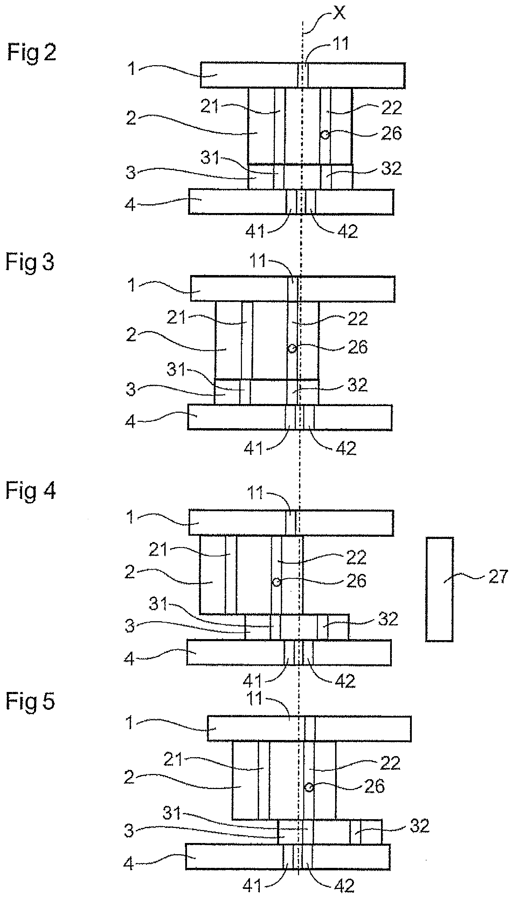

In the second position, see Fig 2, the first fill chamber 21 and the second fill chamber 31 are communicating with each other and are closed to the first communication passage 11 and to the primary channel 41 and the secondary channel 42. The milk sample is thus contained and enclosed in the first fill chamber 21 and the second fill chamber 31, which are aligned to each other.

In the second position, the milk sample contained in the first fill chamber 21 and the second fill chamber 31 may be centrifuged in order to obtain a first sample part of a volume equal to the first volume, and a second sample part of a volume equal to the second volume. The centrifuging may last for 15 to 60 s. The centrifugal force required is estimated to lie in the range 500g to 1800g. After the centrifuging, the first sample part contains all fat and casein, or substantially all fat and casein, of the milk in the milk sample. The second sample part contains all, or substantially all, somatic cells of the milk in the milk sample.

The centrifuging may be performed in a centrifuge (not disclosed) of any suitable kind. The device may be configured to be contained in the centrifuge during the centrifuging of the milk sample.

### Third position

In a third position, see Fig 3, the first communication passage 11, the first mixing chamber 22, the second mixing chamber 32 and the primary channel 41 communicate with each other. The first communication passage 11, the first mixing chamber 22, the second mixing chamber 32 and the primary channel 41 are aligned with each other in the third position, thereby forming a channel extending through the device, in particular in parallel with the longitudinal axis X.

The third position is configured to permit supply of a solution to the first mixing chamber 21 and the second mixing chamber 31. The solution comprises, or consists of, a biologically acceptable saline solution, and may comprise, or consist, of a biological acceptable NaCI-solution. The salt content may then correspond to the salt content of the body liquid of the animal.

During the supply of the solution, the solution is flowing through the first communication passage 11, the first fill chamber 21, the second fill chamber 31 and the primary channel 41. When these are filled with the solution and no gas bubbles are present, the second section 2 and the third section 3 are moved with the aid of the second actuator 24 and the third actuator 34 so that a fourth position is obtained, thereby removing one sample part, i.e. in this example the first sample part, and retaining a remaining sample part, i.e. in this example the second sample part .

### Fourth position

In the fourth position, see Fig 4, the first mixing chamber 22 and the second fill chamber 31 are communicating with each other and are closed to the first communication passage 11 and to the primary channel 41 and the secondary channel 42.

In the fourth position, the first volume of the solution and the remaining sample part, i.e. the second sample part, are mixed in the first mixing chamber 22 and the second fill chamber 31 to form a sample mixture in which the second sample part is uniformly distributed.

The mixing may be performed by means of a stirring member 26, such as a steel ball, contained in the first mixing chamber 22. The stirring member 26 may be moved reciprocally in the first mixing chamber 22 and the second fill chamber 21 by means of a stirring device 27 comprising a moving magnet or magnet field outside the first mixing chamber 22 and the second fill chamber 21 or outside the device. The diameter of the first mixing chamber 22 and the second fill chamber 31 may be somewhat greater than the diameter of the first communication channel 11, the primary channel 41 and the secondary channel 42 in order to maintain the stirring member within the first mixing chamber 22 and the second fill chamber 31.

### Fifth position

In a fifth position, see Fig 5, the first communication passage 11, the first mixing chamber 22, the second fill chamber 31 and the secondary channel 42 communicate with each other. In the fifth position the sample mixture obtained in the fourth position is discharged via an outlet channel 45, see Fig 1. A predetermined portion of the sample mixture may then be transferred to an analysing equipment 46, schematically indicated in Fig 1, configured for counting and analysing the cell content of the sample mixture.

It is to be noted that the abutment of the sections 1, 2, 3 and 4 against each other, which is discussed above, prevents any leakage of milk or the solution during the relative movement of the sections 1, 2, 3 and 4 to the different positions, and also when the sections 1, 2, 3 and 4 are positioned in the different positions.

In the example discussed above, the sample mixture to be discharged from the device and analysed contains the second constituent, i.e. somatic cells. However, it is to be noted that the device is suitable also for isolating the first constituent, i.e. fat cells. In this case, the sections 1, 2, 3 and 4 are the same and are arranged in the same way in the first, second and third positions. However, in order to obtain a first sample part containing only the first constituent, it may be advantageous to adapt the length and the volume of the second fill chamber 31 and the second mixing chamber 32 to the total volume of the first and third constituents after the separation. The fourth position, in which the first fill chamber 21 and the second mixing chamber 32 are communicating with each other and closed to the first communication passage 11 and the second communication passage 43, permits mixing of the solution and the first sample part, in the first fill chamber 21 and the second mixing chamber 32 to form a sample mixture in which the first sample part is distributed. In the fifth position, the first communication passage 11 and the second communication passage 41 communicate with each other and with the first fill chamber 21 and the second mixing chamber 32 to permit discharge of the sample mixture.

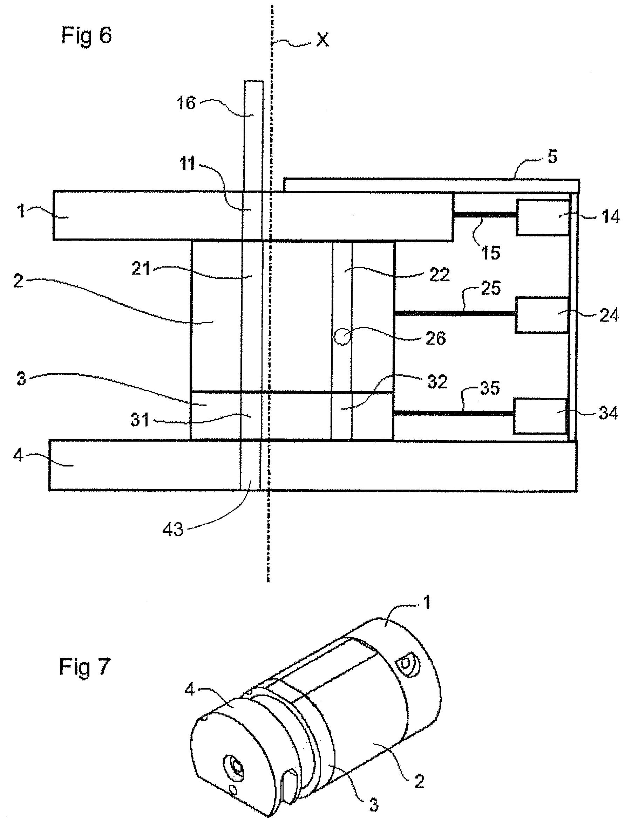

Fig 6 discloses a second embodiment, which differs from the first embodiment in that the second communication passage of the fourth section 4 comprises only one channel 43, which extends through the fourth section 4. The channel 43 will thus operate as inlet or outlet for the milk in the first position, as inlet or outlet for the solution in the third position, and inlet or outlet for sample mixture in the fifth position. In this embodiment, it is possible to let the first section 1 and the fourth section 4 be attached and stationary in relation to the frame 4, wherein the second section 2 and the third section 3 are individually movable with respect to each other, the frame 5 and the first and fourth sections 1 and 4.

It is to be noted that elements having the same or similar functions in the different embodiments have been allotted the same reference signs in all embodiments.

Fig 7 and 8A-13C illustrates a third embodiment. In these figures only the first section 1, the second section 2, the third section 3 and the fourth section 4 are shown. The third embodiment differs from the first embodiment in that the first section 1, the second section 2 and the third section 3 are rotatable around the longitudinal axis X to the different positions described above. The fourth section 4 is stationary with respect to the frame, which is not disclosed in these figures.

In the third embodiment, the first section 1, the second section 2, the third section 3 and the fourth section 4 are held together by means of a bolt 50 extending through a centre hole through all section 1 to 4 along the longitudinal axis X forming a centre axis of the bolt 50. The sections 1 to 4 are pre-tensioned against each other by means of a spring 51. The pre-tensioning force may be regulated by means of one or more nut 52 threaded onto the bolt 50.

No frame is shown in Figs 7 and 8A to 13C, although a frame corresponding to the one of the first and second embodiments may be provided, at least for holding a first actuator for rotating the first section 1, a second actuator for rotating the second section 2 and a third actuator for rotating the third section 3.

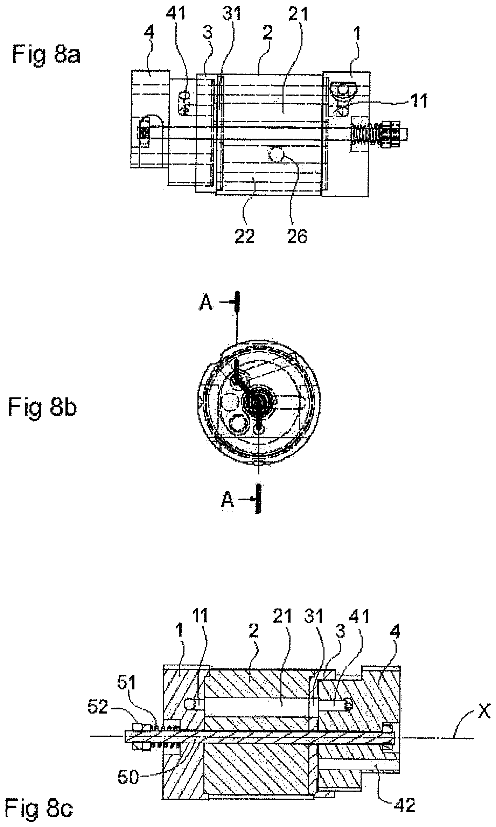

Figs 8A-8C illustrate the device when the sections 1 to 4 are rotated to or positioned in the first position by means of a suitable number of the actuators. In the first position, the first communication passage 11, the first fill chamber 21, the second fill chamber 31 and the primary channel 41 communicate with each other. The first communication passage 11, the first fill chamber 21, the second fill chamber 31 and the second communication passage 43 are arranged after each other to form a channel extending through the device. It can be seen from Figs 8A-8C, that the first communication passage 11 and the primary channel 41 do not extend straight through the first section 1 and fourth section 4, respectively. The first position of the device according to the third embodiment shown in Figs 8A-8C serves the same functions as explained above for the first embodiment.

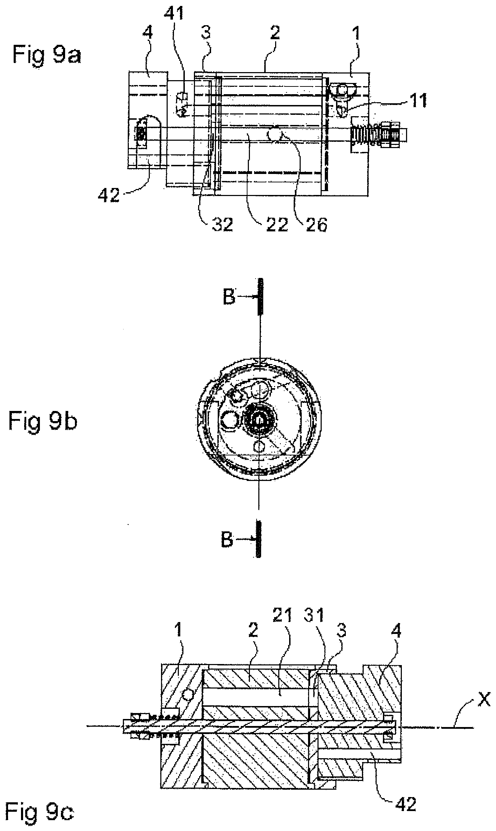

Figs 9A-9C illustrate the device when the sections 1 to 4 are positioned in the second position, i.e. the second section 2 and the third section 3 are rotated in relation to the first section 1 and the fourth section 4 by means of a suitable number of the actuators. In the second position, the first fill chamber 21 and the second fill chamber 31 are communicating with each other and closed to the first communication passage 11 and to the primary channel 41 and the secondary channel 42. The milk sample is thus contained and enclosed in the first fill chamber 21 and the second fill chamber 31, which are aligned to each other. The second position of the device according to the third embodiment shown in Figs 9A-9C serves the same functions as explained above for the first embodiment. The device according the third embodiment is also suitable for being included or integrated in a centrifuge for performing the centrifuging step.

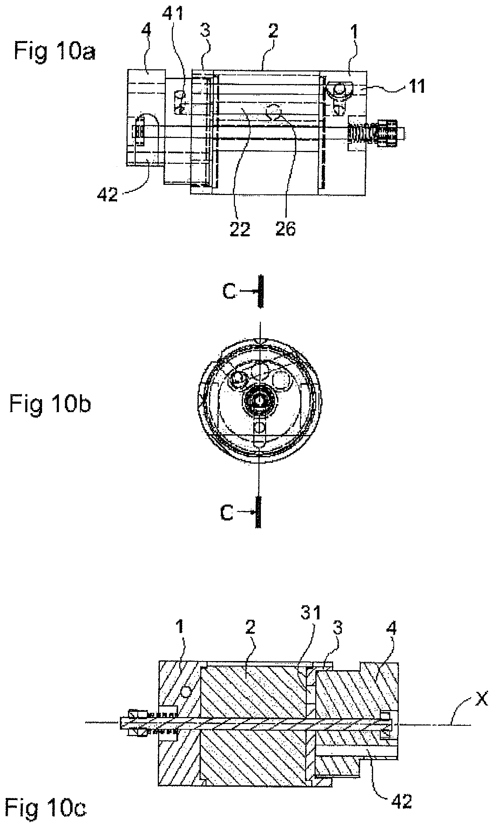

Figs 10A-10C illustrate the device when the sections 1 to 4 are in an intermediate position, in which the third section 3 has been rotated by means of a suitable number of the actuators to isolate the second fill chamber 31 enclosing the second sample part.

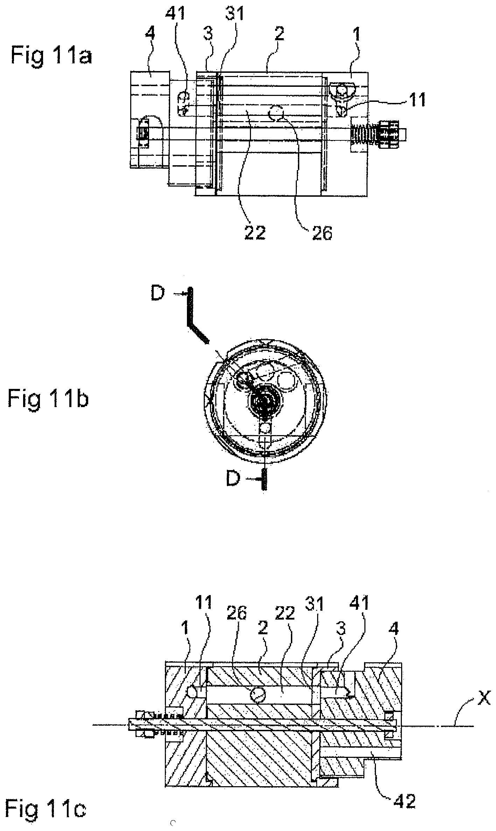

Figs 11A-11C illustrate the device when the sections 1 to 4 are positioned in the third position by means of a suitable number of the actuators. In the third position, the first communication passage 11, the first mixing chamber 22, the second mixing chamber 32 and the primary channel 41 communicate with each other. The first communication passage 11, the first mixing chamber 22, the second mixing chamber 32 and the primary channel 41 are arranged after each other to form a channel extending through the device for the supply of the solution. The third position of the device according to the third embodiment shown in Figs 11A-11C serves the same functions as explained above for the first embodiment.

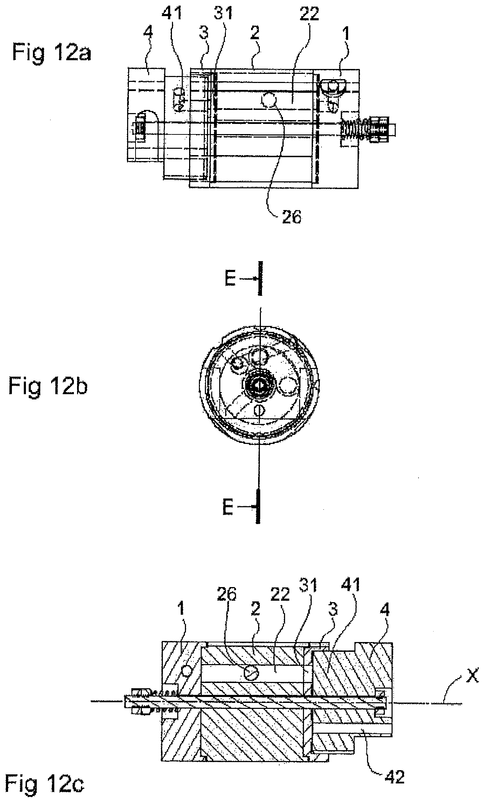

Figs 12A-12C illustrate the device when the sections 1 to 4 are positioned in the fourth position by means of a suitable number of the actuators. In the fourth position, the first mixing chamber 22 and the second fill chamber 31 are communicating with each other and closed to the first communication passage 11 and to the primary channel 41 and the secondary channel 42 to permit mixing of the solution and the second sample part to a sample mixture. The fourth position of the device according to the third embodiment shown in Figs 12A-12C serves the same functions as explained above for the first embodiment.

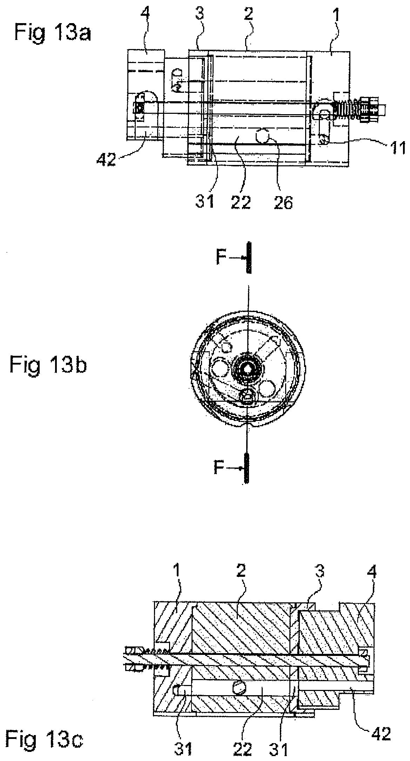

Figs 13A-13C illustrate the device when the sections 1 to 4 are positioned in the fifth position by means of a suitable number of the actuators. In the fifth position, the first communication passage 11, the first mixing chamber 22, the second fill chamber 31 and the secondary channel 42 communicate with each other to permit discharge of the sample mixture. The fifth position of the device according to the third embodiment shown in Figs 13A-13C serves the same functions as explained above for the first embodiment.

In the same way as the first and second embodiments, also the third embodiment is suitable for isolating and discharging a sample mixture containing fat cells.

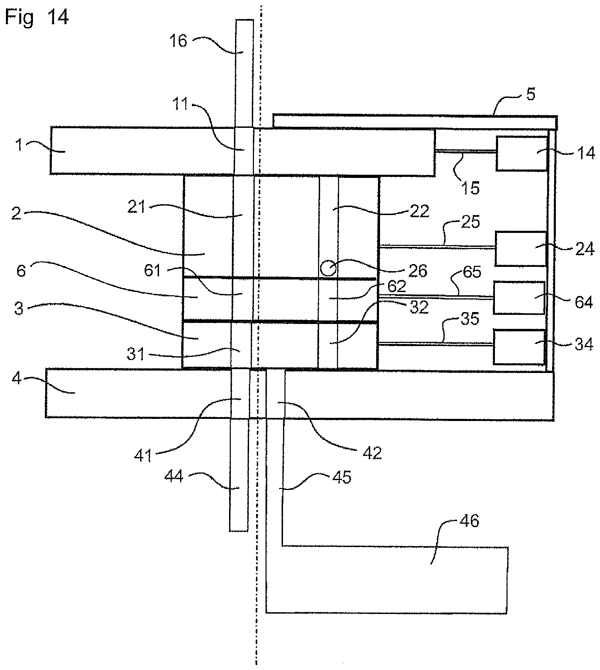

Fig 14 discloses a fourth embodiment of the invention, which corresponds to the device according to the first embodiment described above, but which comprises a fifth section 6 provided between the second section 2 and the third section 3. The fifth section 6 comprises a fifth fill chamber 61 and a fifth mixing chamber 62. The fifth section 6 is movable in the same way as the second and third sections 2 and 3 to be aligned alternatively with the fill chambers 21, 31 and the mixing chambers 22, 32 of the second and third sections 2 and 3. With such a fifth section 6, it is possible to separate the third constituent of the milk. For instance, the third constituent may be casein as mentioned above. The fifth section 6 is movable in the same way as the first, second and third section 1, 2, 3 by means of a fifth actuator 64 attached to the frame 5 and connected to the first section 1 via a schematically indicated first transmission device 65 or transmission element, such as a piston, a gear rack, a pulley etc.

The sections 1, 2, 3, 4 and 6, are configured to permit the following consecutive relative positions.

In a first position, the first communication passage 11, the first fill chamber 21, the second fill chamber 31, the fifth fill chamber 61 and the second communication passage 43 communicate with each other. The first position permits supply of a milk sample containing at least a first constituent, such as fat, a second constituent, such as somatic cells, and a third constituent, such as casein, to the first fill chamber 21, the second fill chamber 31 and the fifth fill chamber 61.

In a second position, the first fill chamber 21, the second fill chamber 31 and the fifth fill chamber 61 are communicating with each other and closed to the first communication passage 11 and the second communication passage 43. The second position permits centrifuging of the milk sample contained in the first fill chamber 21, the second fill chamber 31 and the fifth fill chamber 61 to obtain a first sample part of a first volume in the first fill chamber 21, in which first sample part the first constituent is contained, a second sample part of a second volume in the second fill chamber 31, in which second sample part the second constituent is contained, and a third sample part of a third volume in the fifth fill chamber 61, in which third sample part the third constituent is contained.

In a third position, the first communication passage 11, the first mixing chamber 22, the second mixing chamber 32, the fifth mixing chamber 62 and the second communication passage 43 communicate with each other. The third position permits supply of a solution to the first mixing chamber 21, the second mixing chamber 31 and the fifth mixing chamber 61.

In a fourth position, one of the first, second and fifth fill chambers 21, 31, 61 is communicating with two of the first, second and fifth mixing chambers 22, 32, 62, thereby forming a channel extending through the second, fifth and third sections 2, 6 and 3 and being closed to the first communication passage 11 and the second communication passage 43. The fourth position permits mixing of the solution and either first sample part, the second sample part or the third sample part to form a sample mixture in which this sample part is uniformly distributed.

In a fifth position, the first communication passage and the second communication passage communicate with each other and with said channel to permit discharge of the sample mixture.

It should be noted that not only the first embodiment but also the second and third embodiments may be modified to include five sections as explained above for the fourth embodiment.

The present invention is not limited to the embodiments disclosed but may be varied and modified within the scope of the following claims.

It is also to be noted that the stirring member 26 and the stirring device 27 shown may be replaced by any other stirring equipment. For instance the stirring may be performed by circulating the solution and the second sample part through external channels temporarily connected to the first mixing chamber 22 and the second fill chamber 31 when these are in the fourth position.

## Claims

1. A method for preparing a milk sample, the method comprising the steps of:
providing a milk sample containing at least a first constituent and a second constituent, and
centrifuging the milk sample to obtain a plurality of sample parts including at least a first sample part of a first volume, in which first sample part the first constituent is contained, and a second sample part of a second volume, in which second sample part the second constituent is contained,
providing a solution,
removing at least one of the sample parts from the milk sample, thereby retaining a remaining sample part of the plurality of sample parts,
mixing the remaining sample part and the solution to form a sample mixture in which the remaining sample part is distributed, and
discharging the sample mixture, **characterised by that:**
the step of providing the milk sample is followed by a step of isolating the milk sample in a space formed by a first fill chamber (21), defining the first volume, and a second fill chamber (31), defining of the second volume, and
the step of providing the solution is followed by a step of isolating the solution in a space formed by a first mixing chamber (22), defining a volume equal to the first volume, and a second mixing chamber (32), defining a volume equal to the second volume, and wherein the step of removing one of the sample parts comprises displacing at least one of the second fill chamber (31) and the first mixing camber (22) to communicate with each other.

2. A method according to claim 1, wherein the discharging step comprises transferring a determined portion of the sample mixture to an analysing equipment configured for counting and analysing the cell content of the sample mixture.

3. A device configured to be used when preparing a milk sample, comprising a plurality of sections (1, 2, 3, 4 and 6) including at least
a first section (1) forming a first communication passage (11) extending through the first section (1),
a second section (2) forming a first fill chamber (21), defining a first volume and extending through the second section (2), and a first mixing chamber (22), defining a volume equal to the first volume and extending through the second section (2),
a third section (3) forming a second fill chamber (31), defining a second volume and extending through the third section (3), and a second mixing chamber (32), defining a volume equal to the second volume and extending through the third section (3), and
a fourth section (4) forming a second communication passage (41, 42; 43) extending through the fourth section (4), wherein at least the second section (2) and the third section (3) are individually movable transversely to a longitudinal axis (X) of the device to permit different relative positions of the sections (1, 2, 3, 4 and 6), all the sections (1, 2, 3, 4 and 6) being arranged after each other along the longitudinal axis (X).

4. A device according to claim 3, wherein the second section (2) and the third section (3) are individually movable transversely to the longitudinal axis (X) to said relative positions, which define different communications possibilities between the first communication passage (11), the first fill chamber (21), the first mixing chamber (22), the second fill chamber (31), the second mixing chamber (32), and the second communication passage (41, 42; 43).

5. A device according to claim 4, wherein the first section (1), the second section (2), the third section (3) and the fourth section (4) are configured to permit the following consecutive relative positions:
a first position, in which the first communication passage (11), the first fill chamber (21), the second fill chamber (31) and the second communication passage (43) communicate with each other,
a second position, in which the first fill chamber (21) and the second fill chamber (31) are communicating with each other and closed to the first communication passage (11) and the second communication passage (43),
a third position, in which the first communication passage (11), the first mixing chamber (22), the second mixing chamber (32) and the second communication passage (43) communicate with each other,
a fourth position, in which either the first mixing chamber (22) and the second fill chamber (31) or first fill chamber (21) and the second mixing chamber (32) are communicating with each other and closed to the first communication passage (11) and the second communication passage (43), and
a fifth position, in which the first communication passage (11) and the second communication passage (41) communicate with each other and with either the first mixing chamber (22) and the second fill chamber (31) or first fill chamber (21) and the second mixing chamber (32).

6. A device according to claim 5, wherein
the first position is configured to permit supply of a milk sample containing at least a first constituent and a second constituent to the first fill chamber (21) and the second fill chamber (31),
the second position is configured to permit centrifuging of the milk sample contained in the first fill chamber (21) and the second fill chamber (31) to obtain a first sample part of a first volume, in which first sample part the first constituent is contained, and a second sample part of a second volume, in which second sample part the second constituent is contained,
the third position is configured to permit supply of a solution to the first mixing chamber (21) and the second mixing chamber (31),
the fourth position is configured to permit mixing of the solution and either the second sample part, in the first mixing chamber (22) and the second fill chamber (31) to form a sample mixture in which the second sample part is distributed, or the first sample part, in the first fill chamber (21) and the second mixing chamber (32) to form a sample mixture in which the first sample part is distributed, and
the fifth position is configured to permit discharge of the sample mixture.

7. A device according to claim 6, wherein the second communication passage (43) comprises a primary channel (41) and a secondary channel (42), and wherein the first communication passage (11), the first fill chamber (21) and the second fill chamber (31) communicate with the primary channel (41) in the first position for the supply of the milk sample to the first fill chamber (21) and the second fill chamber (31).

8. A device according to claim 7, wherein the first communication passage (11), the first mixing chamber (22) and the second mixing chamber (32) communicate with the primary channel (41) in the third position for the supply of the solution to the first mixing chamber (22) and the second mixing chamber (32).

9. A device according to any one of claims 7 and 8, wherein the first communication passage (11) and either of the first mixing chamber (22) and the second fill chamber (31) or the first fill chamber (21) and the second mixing chamber (32) communicate with the secondary channel (42) in the fifth position for the discharge of the sample mixture.

10. A device according to any one of claims 3 to 9, wherein the first fill chamber (21) and the second fill chamber (31) extend in parallel with the longitudinal axis (X) of the device, and wherein the first mixing chamber (22) and the second mixing chamber (32) extend in parallel with said longitudinal axis (X).

11. A device according to any one of claims 3 to 10, wherein the first section (1), the second section (2) and the third section (3) are individually movable by being rotatable around the longitudinal axis (X).

12. A device according to any one of claims 10 and 11, wherein a first actuator (14) is provided for moving the first section (1), a second actuator (24) is provided for moving the second section (2), and a third actuator (34) is provided for moving the third section (3)

13. A device according to any one of claims 3 to 12, wherein the plurality of sections (1, 2, 3, 4 and 6) comprises a fifth section (6) provided between the second section (2) and the third section (3), and forming a fifth fill chamber (61), defining a fifth volume, and a fifth mixing chamber (62), defining a volume equal to the fifth volume.

14. A device according to claim 13, wherein the sections (1, 2, 3, 4 and 6) are configured to permit the following consecutive relative positions:
a first position, in which the first communication passage (11), the first fill chamber (21), the second fill chamber (31), the fifth fill chamber (61) and the second communication passage (43) communicate with each other,
a second position, in which the first fill chamber (21), the second fill chamber (31) and the fifth fill chamber (61) are communicating with each other and closed to the first communication passage (11) and the second communication passage (43),
a third position, in which the first communication passage (11), the first mixing chamber (22), the second mixing chamber (32), the fifth mixing chamber (62) and the second communication passage (43) communicate with each other,
a fourth position, in which one of the first, second and fifth fill chambers (21, 31, 61) is communicating with two of the first, second and fifth mixing chambers (22, 32, 62), thereby forming a channel extending through the second, fifth and third sections (2, 5, and 3) and being closed to the first communication passage (11) and the second communication passage (43), and
a fifth position, in which the first communication passage (11) and the second communication passage (41) communicate with each other and with said channel.

## Patentansprüche

1. Verfahren zum Vorbereiten einer Milchprobe, wobei das Verfahren die folgenden Schritte umfasst:
Bereitstellen einer Milchprobe, die wenigstens einen ersten Bestandteil und einen zweiten Bestandteil enthält, und
Zentrifugieren der Milchprobe, um mehrere Probenteile zu erhalten, einschließlich wenigstens eines ersten Probenteils eines ersten Volumens, wobei im ersten Probenteil der erste Bestandteil enthalten ist, und eines zweiten Probenteils eines zweiten Volumens, wobei im zweiten Probenteil der zweite Bestandteil enthalten ist,
Bereitstellen einer Lösung,
Entfernen wenigstens eines der Probenteile von der Milchprobe, dadurch Zurückhalten eines verbleibenden Probenteils der mehreren Probenteile,
Mischen des verbleibenden Probenteils und der Lösung, um ein Probengemisch auszubilden, in dem der verbleibende Probenteil verteilt ist, und
Abgeben des Probengemischs, **dadurch gekennzeichnet, dass:**
der Schritt des Bereitstellens der Milchprobe gefolgt wird von einem Schritt eines Isolierens der Milchprobe in einem Raum, der von einer ersten Füllkammer (21), die das erste Volumen definiert, und einer zweiten Füllkammer (31), die das zweite Volumen definiert, ausgebildet ist, und
der Schritt des Bereitstellens der Lösung gefolgt wird von einem Schritt eines Isolierens der Lösung in einem Raum, der von einer ersten Mischkammer (22), die ein dem ersten Volumen entsprechendes Volumen definiert, und einer zweiten Mischkammer (32), die ein dem zweiten Volumen entsprechendes Volumen definiert, ausgebildet ist, und wobei der Schritt des Entfernens eines der Probenteile ein Verschieben der zweiten Füllkammer (31) und/oder der ersten Mischkammer (22) umfasst, um miteinander in Verbindung zu stehen.

2. Verfahren nach Anspruch 1, wobei der Abgabeschritt ein Übertragen eines bestimmten Abschnitts des Probengemischs in ein Analysegerät, das zum Zählen und Analysieren des Zellgehalts des Probengemischs konfiguriert ist, umfasst.

3. Vorrichtung, die konfiguriert ist, beim Vorbereiten einer Milchprobe verwendet zu werden, mehrere Stücke (1, 2, 3, 4 und 6) umfassend, die wenigstens Folgende enthalten:
ein erstes Stück (1), das einen ersten Verbindungsdurchgang (11) ausbildet, der sich durch das erste Stück (1) hindurch erstreckt,
ein zweites Stück (2), das eine erste Füllkammer (21), die ein erstes Volumen definiert und sich durch das zweite Stück (2) hindurch erstreckt, und eine erste Mischkammer (22), die ein dem ersten Volumen entsprechendes Volumen definiert und sich durch das zweite Stück (2) hindurch erstreckt, ausbildet,
ein drittes Stück (3), das eine zweite Füllkammer (31), die ein zweites Volumen definiert und sich durch das dritte Stück (3) hindurch erstreckt, und eine zweite Mischkammer (32), die ein dem zweiten Volumen entsprechendes Volumen definiert und sich durch das dritte Stück (3) hindurch erstreckt, ausbildet, und
ein viertes Stück (4), das einen zweiten Verbindungsdurchgang (41, 42; 43) ausbildet, der sich durch das vierte Stück (4) erstreckt,
wobei wenigstens das zweite Stück (2) und das dritte Stück (3) schräg zu einer Längsachse (X) der Vorrichtung einzeln bewegbar sind, um verschiedene relative Positionen der Stücke (1, 2, 3, 4 und 6) zu ermöglichen, wobei alle Stücke (1, 2, 3, 4 und 6) nacheinander entlang der Längsachse (X) angeordnet sind.

4. Vorrichtung nach Anspruch 3, wobei das zweite Stück (2) und das dritte Stück (3) schräg zu der Längsachse (X) einzeln in die relativen Positionen bewegbar sind, die verschiedene Verbindungsmöglichkeiten zwischen dem ersten Verbindungsdurchgang (11), der ersten Füllkammer (21), der ersten Mischkammer (22), der zweiten Füllkammer (31), der zweiten Mischkammer (32) und dem zweiten Verbindungsdurchgang (41, 42; 43) definieren.

5. Vorrichtung nach Anspruch 4, wobei das erste Stück (1), das zweite Stück (2), das dritte Stück (3) und das vierte Stück (4) konfiguriert sind, die folgenden aufeinanderfolgenden relativen Positionen zu ermöglichen:
eine erste Position, in der der erste Verbindungsdurchgang (11), die erste Füllkammer (21), die zweite Füllkammer (31) und der zweite Verbindungsdurchgang (43) miteinander in Verbindung stehen,
eine zweite Position, in der die erste Füllkammer (21) und die zweite Füllkammer (31) miteinander in Verbindung stehen und zum ersten Verbindungsdurchgang (11) und zum zweiten Verbindungsdurchgang (43) hin geschlossen sind,
eine dritte Position, in der der erste Verbindungsdurchgang (11), die erste Mischkammer (22), die zweite Mischkammer (32) und der zweite Verbindungsdurchgang (43) miteinander in Verbindung stehen,
eine vierte Position, in der entweder die erste Mischkammer (22) und die zweite Füllkammer (31) oder die erste Füllkammer (21) und die zweite Mischkammer (32) miteinander in Verbindung stehen und zum ersten Verbindungsdurchgang (11) und zum zweiten Verbindungsdurchgang (43) hin geschlossen sind, und
eine fünfte Position, in der der erste Verbindungsdurchgang (11) und der zweite Verbindungsdurchgang (41) miteinander und entweder mit der ersten Mischkammer (22) und der zweiten Füllkammer (31) oder mit der ersten Füllkammer (21) und der zweiten Mischkammer (32) in Verbindung stehen.

6. Vorrichtung nach Anspruch 5, wobei
die erste Position konfiguriert ist, ein Zuführen einer Milchprobe, die wenigstens einen ersten Bestandteil und einen zweiten Bestandteil enthält, zur ersten Füllkammer (21) und zur zweiten Füllkammer (31) zu ermöglichen,
die zweite Position konfiguriert ist, ein Zentrifugieren der Milchprobe, die in der ersten Füllkammer (21) und der zweiten Füllkammer (31) enthalten ist, zu ermöglichen, um einen ersten Probenteil eines ersten Volumens, wobei im ersten Probenteil der erste Bestandteil enthalten ist, und einen zweiten Probenteil eines zweiten Volumens, wobei im zweiten Probenteil der zweite Bestandteil enthalten ist, zu erhalten,
die dritte Position konfiguriert ist, ein Zuführen einer Lösung zur ersten Mischkammer (21) und zur zweiten Mischkammer (31) zu ermöglichen,
die vierte Position konfiguriert ist, ein Mischen der Lösung und entweder des zweiten Probenteils in der ersten Mischkammer (22) und der zweiten Füllkammer (31) zum Ausbilden eines Probengemischs, in dem der zweite Probenteil verteilt ist, oder des ersten Probenteils in der ersten Füllkammer (21) und der zweiten Mischkammer (32) zum Ausbilden eines Probengemischs, in dem der erste Probenteil verteilt ist, zu ermöglichen, und
die fünfte Position konfiguriert ist, ein Abgeben des Probengemischs zu ermöglichen.

7. Vorrichtung nach Anspruch 6, wobei der zweite Verbindungsdurchgang (43) einen primären Kanal (41) und einen sekundären Kanal (42) umfasst und wobei der erste Verbindungsdurchgang (11), die erste Füllkammer (21) und die zweite Füllkammer (31) mit dem primären Kanal (41) in der ersten Position in Verbindung stehen, um die Milchprobe in die erste Füllkammer (21) und die zweite Füllkammer (31) zuzuführen.

8. Vorrichtung nach Anspruch 7, wobei der erste Verbindungsdurchgang (11), die erste Mischkammer (22) und die zweite Mischkammer (32) mit dem primären Kanal (41) in der dritten Position in Verbindung stehen, um die Lösung in die erste Mischkammer (22) und die zweite Mischkammer (32) zuzuführen.

9. Vorrichtung nach einem der Ansprüche 7 und 8, wobei der erste Verbindungsdurchgang (11) und entweder die erste Mischkammer (22) und die zweite Füllkammer (31) oder die erste Füllkammer (21) und die zweite Mischkammer (32) mit dem sekundären Kanal (42) in der fünften Position in Verbindung stehen, um das Probengemisch abzugeben.

10. Vorrichtung nach einem der Ansprüche 3 bis 9, wobei die erste Füllkammer (21) und die zweite Füllkammer (31) sich parallel zur Längsachse (X) der Vorrichtung erstrecken und wobei die erste Mischkammer (22) und die zweite Mischkammer (32) sich parallel zur Längsachse (X) erstrecken.

11. Vorrichtung nach einem der Ansprüche 3 bis 10, wobei das erste Stück (1), das zweite Stück (2) und das dritte Stück (3) einzeln bewegbar sind, indem sie um die Längsachse (X) herum drehbar sind.

12. Vorrichtung nach einem der Ansprüche 10 und 11, wobei ein erster Aktuator (14) zum Bewegen des ersten Stücks (1) bereitgestellt ist, ein zweiter Aktuator (24) zum Bewegen des zweiten Stücks (2) bereitgestellt ist und ein dritter Aktuator (34) zum Bewegen des dritten Stücks (3) bereitgestellt ist.

13. Vorrichtung nach einem der Ansprüche 3 bis 12, wobei die mehreren Stücke (1, 2, 3, 4 und 6) ein fünftes Stück (6) umfassen, das zwischen dem zweiten Stück (2) und dem dritten Stück (3) bereitgestellt ist und eine fünfte Füllkammer (61), die ein fünftes Volumen definiert, und eine fünfte Mischkammer (62), die ein dem fünften Volumen entsprechendes Volumen definiert, ausbildet.

14. Vorrichtung nach Anspruch 13, wobei die Stücke (1, 2, 3, 4 und 6) konfiguriert sind, um die folgenden aufeinanderfolgenden relativen Positionen zu ermöglichen:
eine erste Position, in der der erste Verbindungsdurchgang (11), die erste Füllkammer (21), die zweite Füllkammer (31), die fünfte Füllkammer (61) und der zweite Verbindungsdurchgang (43) miteinander in Verbindung stehen,
eine zweite Position, in der die erste Füllkammer (21), die zweite Füllkammer (31) und die fünfte Füllkammer (61) miteinander in Verbindung stehen und zum ersten Verbindungsdurchgang (11) und zum zweiten Verbindungsdurchgang (43) hin geschlossen sind,
eine dritte Position, in der der erste Verbindungsdurchgang (11), die erste Mischkammer (22), die zweite Mischkammer (32), die fünfte Mischkammer (62) und der zweite Verbindungsdurchgang (43) miteinander in Verbindung stehen,
eine vierte Position, in der eine der ersten, der zweiten und der fünften Füllkammer (21, 31, 61) mit zwei der ersten, der zweiten und der fünften Mischkammer (22, 32, 62) in Verbindung steht, wodurch ein Kanal ausgebildet wird, der sich durch das zweite, das fünfte und das dritte Stück (2, 5 und 3) erstreckt und zum ersten Verbindungsdurchgang (11) und zum zweiten Verbindungsdurchgang (43) hin geschlossen ist, und
eine fünfte Position, in der der erste Verbindungsdurchgang (11) und der zweite Verbindungsdurchgang (41) miteinander und mit dem Kanal in Verbindung stehen.

## Revendications

1. Procédé de préparation d'un échantillon de lait, le procédé comprenant les étapes suivantes :
fournir un échantillon de lait contenant au moins un premier constituant et un deuxième constituant, et
centrifuger l'échantillon de lait pour obtenir une pluralité de parties d'échantillon comprenant au moins une première partie d'échantillon d'un premier volume, le premier constituant étant contenu dans ladite première partie d'échantillon, et une seconde partie d'échantillon d'un second volume, le deuxième constituant étant contenu dans ladite seconde partie d'échantillon,
fournir une solution,
éliminer au moins une des parties d'échantillon de l'échantillon de lait, conservant ainsi une partie d'échantillon restante parmi la pluralité de parties d'échantillon,
mélanger la partie d'échantillon restante et la solution pour former un mélange d'échantillon dans lequel la partie d'échantillon restante est répartie, et
évacuer le mélange d'échantillon, **caractérisé en ce que** :
l'étape de fourniture de l'échantillon de lait est suivie d'une étape d'isolation de l'échantillon de lait dans un espace formé par une première chambre de remplissage (21), définissant le premier volume, et une deuxième chambre de remplissage (31), définissant le second volume, et
l'étape de fourniture de la solution est suivie d'une étape d'isolation de la solution dans un espace formé par une première chambre de mélange (22), définissant un volume égal au premier volume, et une deuxième chambre de mélange (32), définissant un volume égal au second volume, et dans lequel l'étape d'élimination d'une des parties d'échantillon comprend le déplacement d'au moins une chambre parmi la deuxième chambre de remplissage (31) et la première chambre de mélange (22) pour communiquer entre elles.

2. Procédé selon la revendication 1, dans lequel l'étape d'évacuation comprend le transfert d'une partie déterminée du mélange d'échantillon vers un équipement d'analyse conçu pour compter et analyser le contenu cellulaire du mélange d'échantillon.

3. Dispositif conçu pour être utilisé lorsque l'on prépare un échantillon de lait, comprenant une pluralité de sections (1, 2, 3, 4 et 6) comprenant au moins
une première section (1) formant un premier passage de communication (11) traversant la première section (1),
une deuxième section (2) formant une première chambre de remplissage (21), définissant un premier volume et traversant la deuxième section (2), et une première chambre de mélange (22), définissant un volume égal au premier volume et traversant la deuxième section (2),
une troisième section (3) formant une deuxième chambre de remplissage (31), définissant un deuxième volume et traversant la troisième section (3), et une deuxième chambre de mélange (32), définissant un volume égal au deuxième volume et traversant la troisième section (3), et
une quatrième section (4) formant un second passage de communication (41, 42 ; 43) traversant la quatrième section (4), dans lequel au moins la deuxième section (2) et la troisième section (3) peuvent être déplacées transversalement individuellement par rapport à un axe longitudinal (X) du dispositif pour permettre différentes positions relatives des sections (1, 2, 3, 4 et 6), toutes les sections (1, 2, 3, 4 et 6) étant disposées l'une après l'autre le long de l'axe longitudinal (X).

4. Dispositif selon la revendication 3, dans lequel la deuxième section (2) et la troisième section (3) peuvent être déplacées transversalement individuellement par rapport à l'axe longitudinal (X) vers lesdites positions relatives, qui définissent différentes possibilités de communication entre le premier passage de communication (11), la première chambre de remplissage (21), la première chambre de mélange (22), la deuxième chambre de remplissage (31), la deuxième chambre de mélange (32) et le second passage de communication (41, 42 ; 43).

5. Dispositif selon la revendication 4, dans lequel la première section (1), la deuxième section (2), la troisième section (3) et la quatrième section (4) sont conçues pour permettre les positions relatives consécutives suivantes :
une première position, dans laquelle le premier passage de communication (11), la première chambre de remplissage (21), la deuxième chambre de remplissage (31) et le second passage de communication (43) communiquent entre eux,
une deuxième position, dans laquelle la première chambre de remplissage (21) et la deuxième chambre de remplissage (31) communiquent entre elles et ne communiquent pas avec le premier passage de communication (11) et le second passage de communication (43),
une troisième position, dans laquelle le premier passage de communication (11), la première chambre de mélange (22), la deuxième chambre de mélange (32) et le second passage de communication (43) communiquent entre eux,
une quatrième position, dans laquelle soit la première chambre de mélange (22) et la deuxième chambre de remplissage (31) soit la première chambre de remplissage (21) et la deuxième chambre de mélange (32) communiquent entre elles et ne communiquent pas avec le premier passage de communication (11) et le second passage de communication (43), et
une cinquième position, dans laquelle le premier passage de communication (11) et le second passage de communication (41) communiquent entre eux et avec la première chambre de mélange (22) et la deuxième chambre de remplissage (31) ou avec la première chambre de remplissage (21) et la deuxième chambre de mélange (32).

6. Dispositif selon la revendication 5, dans lequel
la première position est conçue pour permettre la fourniture d'un échantillon de lait contenant au moins un premier constituant et un deuxième constituant à la première chambre de remplissage (21) et à la deuxième chambre de remplissage (31),
la deuxième position est conçue pour permettre la centrifugation de l'échantillon de lait contenu dans la première chambre de remplissage (21) et la deuxième chambre de remplissage (31) pour obtenir une première partie d'échantillon d'un premier volume, le premier constituant étant contenu dans ladite première partie d'échantillon, et une deuxième partie d'échantillon d'un second volume, le deuxième constituant étant contenu dans ladite seconde partie d'échantillon,
la troisième position est conçue pour permettre la fourniture d'une solution à la première chambre de mélange (21) et à la deuxième chambre de mélange (31),
la quatrième position est conçue pour permettre le mélange de la solution et soit de la deuxième partie d'échantillon, dans la première chambre de mélange (22) et la deuxième chambre de remplissage (31) pour former un mélange d'échantillon dans lequel la deuxième partie d'échantillon est répartie, soit de la première partie d'échantillon, dans la première chambre de remplissage (21) et la deuxième chambre de mélange (32) pour former un mélange d'échantillon dans lequel la première partie d'échantillon est répartie, et
la cinquième position est conçue pour permettre l'évacuation du mélange d'échantillon.

7. Dispositif selon la revendication 6, dans lequel le second passage de communication (43) comprend un canal primaire (41) et un canal secondaire (42), et dans lequel le premier passage de communication (11), la première chambre de remplissage (21) et la deuxième chambre de remplissage (31) communiquent avec le canal primaire (41) dans la première position pour la fourniture de l'échantillon de lait dans la première chambre de remplissage (21) et la deuxième chambre de remplissage (31).

8. Dispositif selon la revendication 7, dans lequel le premier passage de communication (11), la première chambre de mélange (22) et la deuxième chambre de mélange (32) communiquent avec le canal primaire (41) dans la troisième position pour la fourniture de la solution à la première chambre de mélange (22) et à la deuxième chambre de mélange (32).

9. Dispositif selon l'une quelconque des revendications 7 et 8, dans lequel le premier passage de communication (11) et soit la première chambre de mélange (22) et la deuxième chambre de remplissage (31) soit la première chambre de remplissage (21) et la deuxième chambre de mélange (32) communiquent avec le canal secondaire (42) dans la cinquième position pour l'évacuation du mélange d'échantillon.

10. Dispositif selon l'une quelconque des revendications 3 à 9, dans lequel la première chambre de remplissage (21) et la deuxième chambre de remplissage (31) s'étendent parallèlement à l'axe longitudinal (X) du dispositif, et dans lequel la première chambre de mélange (22) et la deuxième chambre de mélange (32) s'étendent parallèlement audit axe longitudinal (X).

11. Dispositif selon l'une quelconque des revendications 3 à 10, dans lequel la première section (1), la deuxième section (2) et la troisième section (3) peuvent être déplacées individuellement en pouvant tourner autour de l'axe longitudinal (X).

12. Dispositif selon l'une quelconque des revendications 10 et 11, dans lequel un premier actionneur (14) est prévu pour déplacer la première section (1), un deuxième actionneur (24) est prévu pour déplacer la deuxième section (2) et un troisième actionneur (34) est prévu pour déplacer la troisième section (3)

13. Dispositif selon l'une quelconque des revendications 3 à 12, dans lequel la pluralité de sections (1, 2, 3, 4 et 6) comprend une cinquième section (6) prévue entre la deuxième section (2) et la troisième section (3) et formant une cinquième chambre de remplissage (61), définissant un cinquième volume, et une cinquième chambre de mélange (62), définissant un volume égal au cinquième volume.

14. Dispositif selon la revendication 13, dans lequel les sections (1, 2, 3, 4 et 6) sont conçues pour permettre les positions relatives consécutives suivantes :
une première position, dans laquelle le premier passage de communication (11), la première chambre de remplissage (21), la deuxième chambre de remplissage (31), la cinquième chambre de remplissage (61) et le second passage de communication (43) communiquent entre eux,
une deuxième position, dans laquelle la première chambre de remplissage (21), la deuxième chambre de remplissage (31) et la cinquième chambre de remplissage (61) communiquent entre elles et ne communiquent pas avec le premier passage de communication (11) et le second passage de communication (43),
une troisième position, dans laquelle le premier passage de communication (11), la première chambre de mélange (22), la deuxième chambre de mélange (32), la cinquième chambre de mélange (62) et le second passage de communication (43) communiquent entre eux,
une quatrième position, dans laquelle une chambre parmi les première, deuxième et cinquième chambres de remplissage (21, 31, 61) communique avec deux chambres parmi les première, deuxième et cinquième chambres de mélange (22, 32, 62), formant ainsi un canal traversant les deuxième, cinquième et troisième sections (2, 5 et 3) et ne communiquant pas avec le premier passage de communication (11) et le second passage de communication (43), et
une cinquième position, dans laquelle le premier passage de communication (11) et le second passage de communication (41) communiquent l'un avec l'autre et avec ledit canal.
